# EUROPEAN PATENT APPLICATION

(11) **EP 2 775 193 A2**
(43) Date of publication of application: **10.09.2014**
(21) Application number: 14156676.0
(22) Date of filing: 25.02.2014
(51) Int. Cl.: F16P 1/06

(54) **Electric-arc resistant face shield or lens including ir-blocking inorganic nanoparticles**

(30) Priority: 05.03.2013 US 201313785147
(71) Applicant: Honeywell International Inc., Morristown, NJ 07962-2245 (US)
(72) Inventor: Boulton, Jonathan, Morristown, NJ New Jersey 07962-2245 (US); Johnson, Philip, Morristown, NJ New Jersey 07962-2245 (US)
(74) Representative: Houghton, Mark Phillip

(57) **Abstract**

A transparent electric-arc resistant composition is produced by preparing crystalline tungsten bronze nanoparticles and homogenously dispersing the nanoparticles in a transparent plastic matrix. The crystalline tungsten bronze nanoparticles are prepared by homogeneously mixing an aqueous solution of soluble tungsten and cesium salts and then drying the solution. The dried solution is then heated in a reducing gas or inert gas atmosphere to form crystalline tungsten bronze nanoparticles. Thereafter, a dispersion agent is added and the nanoparticles are dispersed in a liquid solvent medium by wet milling to form a dispersion mixture. The dispersion mixture is then mixed with polymer pellets to form a polymer mixture, and the polymer mixture is then extruded to yield polymer pellets containing highly homogeneously dispersed inorganic nanoparticles. During or after dispersion, additional particles, dyes, heat stabilizers or UV absorbers, may be added. A lens or shield (10) is then molded from the polymer pellets.

## Description

### BACKGROUND

The present disclosure relates to electric-arc resistant materials and more particularly to electric-arc resistant face shields.

Electrical workers are at risk from electric arcs or flashes from high voltage equipment. Electric arcs are extremely dangerous and the resulting high temperatures can cause injury through burning and can even cause immediate fatal bums. Due to the dangers involved, personal protective equipment is needed for electric arc protection, including helmets and headgear.

### [03] SUMMARY OF THE DISCLOSURE

The present disclosure relates to electric-arc resistant face shields incorporating IR-blocking nanoparticles.

A transparent plastic composition resistant to an electric arc includes a transparent substrate material and inorganic nanoparticles. The inorganic nanoparticles are dispersed in the substrate so that the composition is substantially transparent. The substrate may be a cellulose-derived material such as cellulose acetate propionate or may be another transparent plastic such as polyester, polycarbonate, polysulfone or polyimides. The nanoparticles preferably comprise tungsten or a tungsten bronze alloy.

The electric-arc resistant plastic composition is produced by preparing crystalline tungsten bronze nanoparticles and homogenously dispersing the nanoparticles in a transparent polymer material. More specifically, the crystalline tungsten bronze nanoparticles are prepared by homogeneously mixing an aqueous solution of soluble tungsten and cesium salts and then drying the solution. The dried solution is then heated in a reducing gas or inert gas atmosphere to form crystalline tungsten bronze nanoparticles. Thereafter, a dispersion agent is added and the nanoparticles are dispersed in a liquid solvent medium by wet milling with small diameter grinding media to form a dispersion mixture. The dispersion mixture is then mixed with polymer pellets to form a polymer mixture, and the polymer mixture is then extruded to yield polymer pellets containing highly homogeneously dispersed inorganic nanoparticles. During or after dispersion, additional particles, dyes, heat stabilizers or UV absorbers, may be added.

In one embodiment, the tungsten bronze powder is Cs_{0.33}WO₃, and the Cs_{0.33}WO₃ nanoparticles are up to 0.2% of the resulting composition, by weight.

A lens or shield molded from the extruded and pelletized polymer material may be used in a face shield or other eye safety products.

BRIEF DESCRIPTION OF THE DRAWINGS

The preferred embodiment will now be described further by way of example with reference to the following examples and figures, which are intended to be illustrative only and in no way limiting upon the scope of the disclosure.

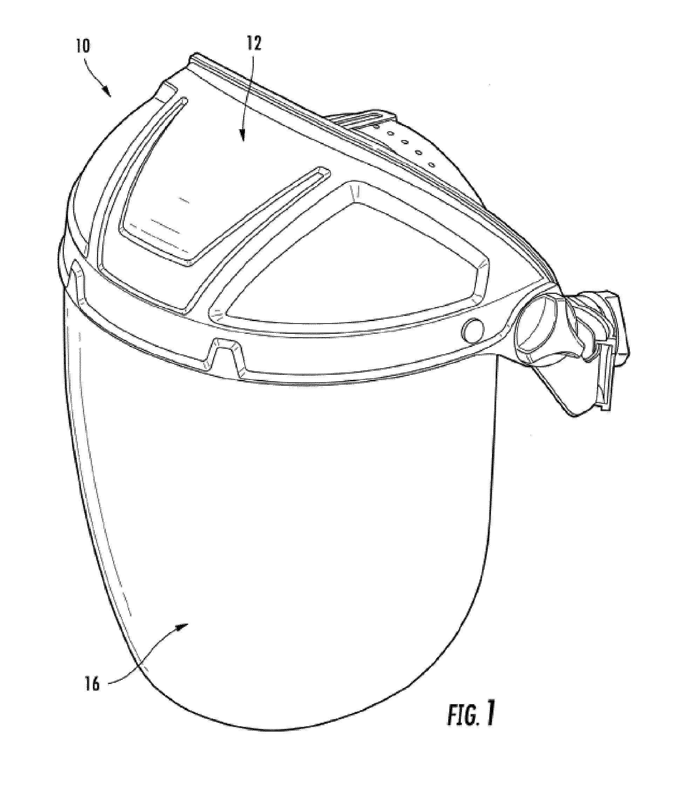

Fig. 1 is an illustration of a face shield including a lens including the composition of the present disclosure.

### [12] DETAILED DESCRIPTION OF THE INVENTION

The present disclosure relates to an electric-arc resistant face shield incorporating IR-blocking nanoparticles.

A transparent composition resistant to an electric arc includes a transparent substrate material and inorganic nanoparticles. The inorganic nanoparticles are dispersed in the substrate so that the composition remains substantially transparent. The substrate may be a cellulose-derived material such as cellulose acetate propionate, or may be another transparent plastic such as polyester, polycarbonate, polysulfone or polyimide. The inorganic nanoparticles preferably comprise tungsten or a tungsten bronze alloy.

The present disclosure describes the use of non-organic pigment based IR blocking materials having higher thermal stability and low visible coloration as a beneficial substitute for low thermal stability dye-based IR absorbers. For example, doped indium tin oxide (ITO) and many other nano-sized conducting metals and black compounds can behave as near infrared (NIR) blocking materials. As further examples, gold, ruthenium dioxides, rhenium trioxides and lanthanum hexaborides can behave as NIR blocking materials.

In a particular exemplary embodiment as described herein, nano-sized reduced tungsten oxide based materials are preferred because they are notably strong and exhibit wide-band NIR absorption coupled with high visible light transmission.

Stoichiometric WO₃ (tungsten oxide) nanoparticles are highly transparent in both the visible and NIR regions. Metallic conductivity and strong IR absorption are induced by reduction or by doping the WO₃. Nanoparticle tungsten bronzes MxWO₃ have strong NIR absorbance and high transmittance in the visible range. A preferred material would be Cs_{0.33}WO₃ due to its higher stability compared to other tungsten-based materials.

Tungsten oxide-based nanoparticles could be utilized in different ways to provide improved electric arc resistance to an article, such as a face shield lens. They could be incorporated as a component of a solution-derived coating applied directly on the lens or they could be directly incorporated into the article itself.

A preferred method would be to incorporate these nano-particles into the bulk matrix of the lens itself. Generally, the article would be made of a formable polymer such as a cellulose derived material, e.g. a cellulose acetate propionate, or another polymer such as polyester, or polycarbonate. High heat resistant polymers, such as polysulfone and polyimides would also be suitable.

A polymer containing such tungsten-based nanoparticles could then be formed by processing methods such as extrusion, typically into a flat sheet from which lens shapes could be die-cut, or injection molded into a flat or curved face shield lens.

Referring to Figure 1, there is shown a protective face shield assembly 10 comprising a crown 12 and an arcuate transparent lens 16. The face shield 10 can be placed on a user's head, such that the lens 16 is supported in front of a user's eyes. The lens 16 includes dispersed nanoparticles in a transparent plastic, and can be made by any of the methods of this disclosure.

Dispersing such nanoparticles into transparent plastics which are then used to produce electric-arc resistant face shields would allow greater design and manufacturing flexibility compared to plastics containing broadband IR dyes. High heat stability would open up the possibility of using other transparent plastics such as high-heat resistant polymers. For example, polycarbonate, polyesters, and polyimides would provide higher thermal stability. High heat stability would also reduce manufacturing complications and limitations due to dye degradation occurring during molding. Without an inherent dark green coloration in the visible region, it would also be possible to create plastics with tailored coloration for enhanced color recognition and tinting.

The starting inorganic nanoparticles are used as a concentrated dispersion in solvent. The mixture would contain dispersion aids and be processed by milling. The dispersion mixture would then be mixed with a polymer (e.g. polycarbonate) pellets and extruded to yield polymer pellets containing highly homogeneously dispersed inorganic nanoparticles. The amount of nanoparticles would be adjusted to ensure the required degree of IR blocking and arc rating in the final product. Other additives, dyes, and pigments could also be incorporated. Good dispersion would be required to ensure low haze in the final part. The pellets would then be molded or extruded to give a sheet or molded into the final shape.

Tungsten oxide-based nanoparticles can be prepared by several methods. Many tungstate compositions would be suitable for blocking IR and use in the present invention but those incorporating cesium would be preferred, alone or in combination with other monovalent or divalent metal ions.

### [25] Example 1

Cs_{0.33}WO₃ tungsten bronze powder can be prepared by homogeneously mixing an aqueous solution of soluble tungsten and cesium salts, e.g. ammonium tungstate and cesium acetate, followed by drying and high temperature treatment in a reducing/inert gas atmosphere to form the final crystalline tungsten bronze nanoparticles.

A homogenous dispersion of the tungsten nanoparticles are then prepared in a liquid solvent medium, e.g. toluene, using a wet milling method with small diameter grinding media. Typically the milling media would be zirconia-based and have a diameter of less than 0.3mm. A horizontal wet media mill would be suitable for this application. A dispersing agent, e.g. a polyacrylate, would be typically added at this stage to improve the dispersion of the powder material and to yield a stable solvent dispersion of the nanoparticle. Other materials including inorganic particles, organic dyes, heat stabilizers and UV absorbers can be added at this stage or later.

The amount or concentration of nanoparticles in the final article would be adjusted to ensure the required degree of IR blocking and arc rating in the final product. Relatively low concentrations of tungsten oxide nanoparticles would be used for this application, for example 0.2% by weight and lower.

After obtaining a good dispersion of the tungstate nanopowder, it is compounded into a polymer by conventional methods, for example, by kneading or by extrusion. Compounding using a twin-screw extruder followed by pelletizing would be the preferred method.

The compounded pelletized material is then molded into a face shield lens by conventional injection molding.

### [31] Example 2

Even lower concentrations of tungsten oxide nanoparticles could be used for this application, for example 0.1 % by weight and lower.

The present disclosure is believed to represent a significant advancement in the art, which has substantial commercial merit.

While there is shown and described herein certain specific structure embodying a composition including inorganic nanoparticles and specific steps for a method of manufacturing the composition, it will be manifest to those skilled in the art that various modifications and rearrangements of the parts or steps may be made without departing from the spirit and scope of the underlying concept and that the same is not limited to the particular forms herein shown and described except insofar as indicated by the scope of the appended claims.

## Claims

1. An electric arc resistant lens for safety eyewear comprising:
a transparent polymer material; and
inorganic nanoparticles dispersed within the polymer material so that the composition is substantially transparent.

2. The lens of claim 1, wherein the polymer material is selected from the group consisting of a cellulosic polymer, polyester, polycarbonate, polysulfone and polyimide.

3. The lens of claim 1, wherein the nanoparticles comprise tungsten.

4. The lens of claim 3, wherein the nanoparticles are crystalline tungsten bronze.

5. The lens of claim 4, wherein the nanoparticles are Cs_{0.33}WO₃.

6. The lens of claim 4, wherein the nanoparticles are up to 0.2% of the composition, by weight.

7. The lens of claim 2, wherein the nanoparticles comprise tungsten.

8. The lens of claim 7, wherein the nanoparticles are crystalline tungsten bronze.

9. The lens of claim 8, wherein the nanoparticles are Cs_{0.33}WO₃.

10. The lens of claim 8, wherein the nanoparticles are up to 0.2% of the composition, by weight.
